# EUROPEAN PATENT APPLICATION

(11) **EP 4 023 470 A1**
(43) Date of publication of application: **06.07.2022**
(21) Application number: 21218276.0
(22) Date of filing: 30.12.2021
(51) Int. Cl.: B60H 1/00, B60H 3/00, B60H 3/06, A61L 9/14

(54) **TRANSIT VEHICLE HVAC SYSTEM WITH SANITIZATION**

(30) Priority: 30.12.2020 US 202063132065 P
(71) Applicant: Thermo King Corporation, Minneapolis, MN 55420-3693 (US)
(72) Inventor: CARR, Jody, Minneapolis, Minnesota, 55420 (US)
(74) Representative: Haseltine Lake Kempner LLP

(57) **Abstract**

An airflow distribution system (200) of an HVAC system for a transit vehicle, including one or more of: a pre-filter (210) including an anti-pathogen material and being installed upstream of a filter (215), a filter (215) including or coated with an anti-pathogen material, and a treatment dispenser (220) providing a treatment to the air, including an anti-pathogen material, to remove pathogens from the air.

## Description

### Field

This disclosure is directed to heating, ventilation, and air conditioning (HVAC) systems for transit vehicles, particularly including additional sanitization of the air passing through the system.

### Background

Transit vehicles such as buses include heating, ventilation, and air conditioning (HVAC) systems to treat the air within the vehicle to provide comfortable temperatures and safe air conditions.

Transit vehicles typically service many riders in a day and have people regularly entering or leaving the vehicle. The passengers can be in close proximity, within an enclosed space in the transit vehicle. These conditions can facilitate the spread of communicable diseases such as COVID-19. Typically, transit vehicles are cleaned using surface cleaning such as wiping down surfaces. This is most frequently performed prior to service or following service.

### Summary

This disclosure is directed to heating, ventilation, and air conditioning (HVAC) systems for transit vehicles, particularly including additional sanitization of the air passing through the system.

The additional sanitization can include one or more of additional filters, filter treatments, addition of cleaners to the airflow, and changes to the direction of airflow in the HVAC system to improve the removal or deactivation of pathogens and optionally to provide air in a pattern that can mitigate the risks faced by those facing the greatest potential exposure such as drivers.

In an embodiment, an airflow distribution system of a heating, ventilation, and air conditioning (HVAC) system for a transit vehicle includes a return air duct configured to receive air from an internal space of the transit vehicle, a filter configured to remove particulate matter from air passing through the filter, at least one sanitizer configured to remove pathogens from air passing through the airflow distribution system, a heat exchanger disposed along a path taken by the air passing through the airflow distribution system, and an air distribution duct configured to convey the air passing through the airflow distribution system to the interior space of the transit vehicle.

In an embodiment, the airflow distribution system further includes a heater configured to heat air passing through the airflow distribution system.

In an embodiment, the air distribution duct is configured to provide the air passing through the HVAC system first to a driver section of the internal space of the transit vehicle.

In an embodiment, the sanitizer includes one or more selected from a pre-filter located upstream of the filter, an anti-pathogen material included in or coated on the filter, and a treatment dispenser configured to provide a treatment to air passing through the HVAC system.

In an embodiment, the airflow distribution system includes the pre-filter, and the pre-filter includes the anti-pathogen material. In an embodiment, the airflow distribution system includes the treatment dispenser, and the treatment dispenser is located between the return air duct and the filter with respect to a flow of the air passing through the airflow distribution system. In an embodiment, the airflow distribution system includes the treatment dispenser, and the treatment dispenser is located between the heat exchanger and the air distribution duct with respect to a flow of the air passing through the airflow distribution system. In an embodiment, the airflow distribution system includes the treatment dispenser, and the treatment further includes one or more of an air freshener, a deodorant, a scented material, or a cleaner for the heat exchanger.

In an embodiment, the airflow distribution system includes two or more of the sanitizers.

In an embodiment, a method of operating an airflow distribution of a heating, ventilation, and air conditioning (HVAC) system for a transit vehicle includes receiving return air, reducing pathogens in air passing through the airflow distribution system including the return air, and distributing the air passing through the airflow distribution system to an internal space of the transit vehicle.

In an embodiment, distributing the air passing through the airflow distribution system to a climate controlled space of the transit vehicle includes providing the air passing through the airflow distribution system first to a driver section of the internal space of the transit vehicle.

In an embodiment, the reducing pathogens includes one or more of filtering the air passing through the HVAC system using a pre-filter upstream of a filter of the HVAC system, filtering the air passing through the HVAC system using the filter of the HVAC system that includes or is treated with an anti-pathogen material, and providing a treatment to the air passing through the HVAC system, where the treatment includes an anti-pathogen material.

In an embodiment, the reducing pathogens includes filtering the air passing through the airflow distribution system using the pre-filter, and the pre-filter includes the anti-pathogen material. In an embodiment, the reducing pathogens includes providing the treatment to the air passing through the airflow distribution system, and the treatment further includes one or more of an air freshener, a deodorant, a scented material, or a cleaner for an HVAC system heat exchanger.

In an embodiment, the reducing pathogens includes two or more of filtering the air passing through the airflow distribution system using a pre-filter upstream of a filter of the airflow distribution system, filtering the air passing through the airflow distribution system using the filter of the airflow distribution system, wherein the filter of the airflow distribution system includes or is treated with an anti-pathogen material, and providing a treatment to the air passing through the airflow distribution system, wherein the treatment includes an anti-pathogen material.

### Drawings

Figure 1 shows a perspective view of a transit vehicle according to an embodiment.
Figure 2 shows a schematic view of an airflow distribution system of a transit vehicle heating, ventilation, and air conditioning (HVAC) system according to an embodiment.
Figure 3 shows a flowchart of a method for controlling pathogen exposure in a transit vehicle.

### Detailed Description

This disclosure is directed to heating, ventilation, and air conditioning (HVAC) systems for transit vehicles, particularly including additional sanitization of the air passing through the system.

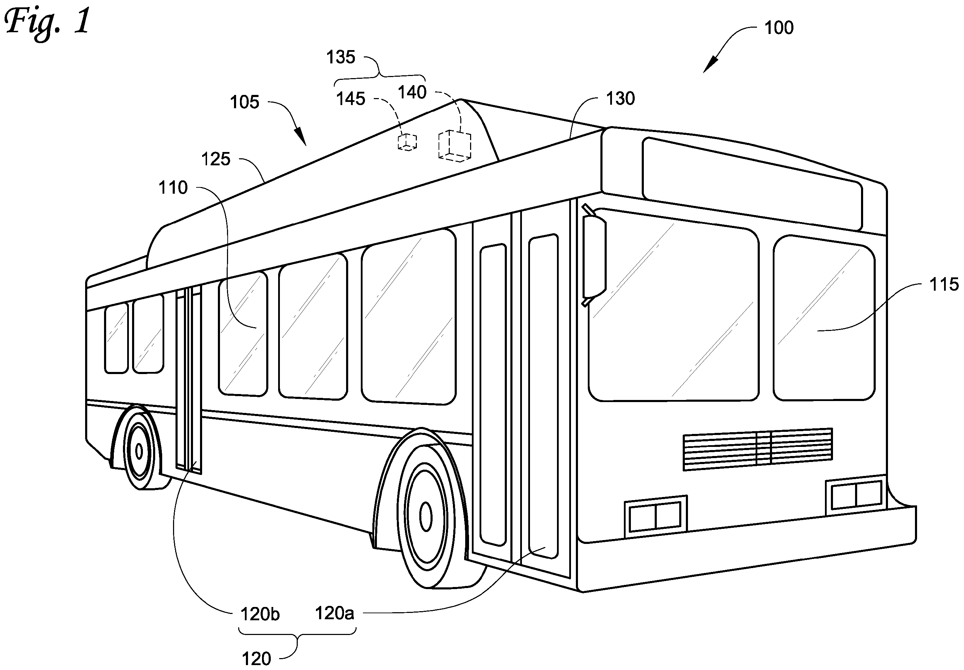

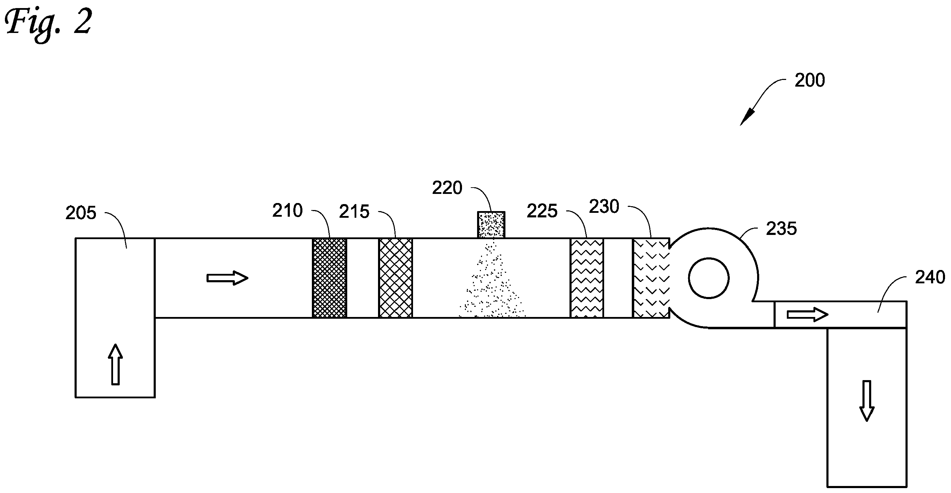

Figure 1 shows a perspective view of a transit vehicle according to an embodiment. Transit vehicle 100 includes a transport climate control system 105, according to one embodiment. While the embodiment shown in Figure 1 is a passenger bus, it is understood that transit vehicles include any vehicle configured to convey multiple passengers over a route. Transit vehicles can include, for example, mass transit buses, school buses, railway vehicles such as passenger cars, light rail transit cars, subway cars, or any other commercial vehicle that can carry passengers to one or more destinations. The transit vehicle 100 includes a climate controlled space 110, such as, as a nonlimiting example, a passenger compartment that can accommodate a plurality of passengers. In an embodiment, the climate controlled space 110 includes a driver section 115. In an embodiment, the driver section 115 is a portion of a passenger compartment. In an embodiment, the driver section 115 is a separate compartment or otherwise separated from the passenger compartment. The transit vehicle 100 includes doors 120 that are positioned on a side of the transit vehicle 100. In the embodiment shown in Fig. 1, a first door 120a is located adjacent to a forward end of the transit vehicle 100, and a second door 120b is positioned towards a rearward end of the transit vehicle 100. Each door 120, such as first door 120a and second door 120b are movable between an open position and a closed position to selectively allow access to the climate controlled space 110. The transport climate control system 105 shown in Figure 1 is a transit vehicle heating, ventilation, and air conditioning (HVAC) system that includes a climate control unit (CCU) 125 attached to a roof 130 of the transit vehicle 100. The transport climate control system 105 can also include an airflow distribution system (see Figure 2) configured to distributed conditioned air within the climate controlled space 110.

The CCU 125 can include a climate control circuit that directs a working fluid (e.g., refrigerant) through, for example, a compressor, a condenser, an evaporator and an expansion device to provide conditioned air within the climate controlled space 110. CCU 125 also includes one or more sanitizers to clean air passing through, reduce pathogens in the air passing through, or otherwise improve air quality and/or reduce pathogen risk within climate controlled space 110. The sanitizers can be one or more of a filter, an auxiliary filter, and a treatment dispenser, as shown in Figure 2 and described below. CCU 125 can further be configured such that air passing from the CCU 125 into climate controlled space 110 passes first to a driver in the driver section 115. The transport climate control system 105 also includes a programmable climate controller 135 and one or more sensors (not shown) that are configured to measure one or more parameters of the transport climate control system 105 (e.g., an ambient temperature outside of the vehicle 100, a space temperature within the climate controlled space 110 an ambient humidity outside of the vehicle 100, a space humidity within the climate controlled space 110, pathogen levels, number of persons entering or leaving the vehicle, vehicle occupancy, airflow within the climate controlled space 110, etc.) and communicate parameter data to the climate controller 135. The climate controller 135 is configured to control operation of the transport climate control system 105 including components of the CCU 125. The climate controller 135 may comprise a single integrated control unit 140 or may comprise a distributed network of climate controller elements 140, 145. The number of distributed control elements in a given network can depend upon the particular application of the principles described herein.

Figure 2 shows a schematic view of an airflow distribution system 200 of a transit vehicle HVAC system (e.g., the transport climate control system 105 shown in Figure 1) according to an embodiment. The airflow distribution system 200 includes return air duct 205, optional auxiliary filter 210, filter 215, treatment dispenser 220, heat exchanger 225, heater 230, blower 235, and air distribution duct 240.

Return air duct 205 is a duct bringing air from within a climate controlled space (e.g., the climate controlled space 110 shown in Figure 1) within the transit vehicle into the airflow distribution system 200. Return air duct 205 is configured to receive air from one or more points within the climate controlled space and to direct the air to optional auxiliary filter 210 or filter 215.

Optional auxiliary filter 210 can be included in embodiments. Optional auxiliary filter 210 can be positioned within the airflow distribution system 200 such that it is readily accessible for servicing or replacement, for example being positioned such that it can be serviced or replaced by a person within the passenger compartment of the transit vehicle. The optional auxiliary filter 210 may have a more frequent replacement schedule compared to filter 215. The optional auxiliary filter 210 can be a filter having a shorter effective life span than that of filter 215. Air passing through both optional auxiliary filter 210 and filter 215 can have lower levels of particulate compared to air that passes only through filter 215 by itself. Air passing through optional auxiliary filter 210 and filter 215 can have lower levels of pathogens when compared to air that passes only through filter 215 by itself. In an embodiment, the auxiliary filter 210 is more selective than filter 215. In an embodiment, auxiliary filter 210 is a specialized filter for removing pathogens such as bacteria and/or viruses from air passing through. In an embodiment, auxiliary filter 210 includes or is treated with an anti-pathogen material. The anti-pathogen material can include one or more of any suitable materials for destroying or deactivating pathogens. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, Environmental Protection Agency (EPA). The anti-pathogen material can be a material included in an amount deemed safe for use where it may be inhaled, for example by selecting the material and/or the amount of the material based on materials safety standards such as those promulgated by the EPA or any other such suitable standard. In an embodiment, the anti-pathogen material is provided as a coating on the auxiliary filter 210. In an embodiment, the auxiliary filter 210 includes or is impregnated with the anti-pathogen material. In an embodiment, the anti-pathogen material may deteriorate or be consumed over time, and a replacement schedule for the auxiliary filter may be based on the effectiveness of the anti-pathogen material over time.

Filter 215 is a filter that air passes through as it travels through the airflow distribution system 200. Filter 215 can be any suitable filter for use in the airflow distribution system 200 to capture particulate matter, absorb or otherwise reduce levels of volatile organic compounds (VOCs) and the like. Filter 215 can be a replaceable filter. In an embodiment, filter 215 includes or is treated with an anti-pathogen material. The anti-pathogen material can include one or more of any suitable materials for destroying or deactivating pathogens. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, the Environmental Protection Agency (EPA). The anti-pathogen material can be a material included in an amount deemed safe for use where it may be inhaled, for example by selecting the material and/or the amount of the material based on materials safety standards such as those promulgated by the EPA or any other such suitable standard. In an embodiment, the anti-pathogen material is provided as a coating on the filter 215. In an embodiment, the filter 215 includes or is impregnated with the anti-pathogen material.

Treatment dispenser 220 can optionally be included in the airflow distribution system 200. Treatment dispenser 220 can be located at any point along the airflow distribution system 200 such that the treatment dispenser can provide a treatment to air passing through the airflow distribution system 200. In an embodiment, treatment dispenser 220 is located at or immediately downstream of return air duct 205. In an embodiment, treatment dispenser 220 is located immediately upstream of or within air distribution duct 240. Treatment dispenser 220 can be a device configured to apply a treatment to air passing through the airflow distribution system 200. The treatment can be, for example, one or more chemicals selected to treat the air as said air passes through the airflow distribution system 200. The treatment can include air fresheners, anti-microbial or anti-pathogen agents, deodorants, scented materials such as aromatherapy scents, cleaners for HVAC system heat exchangers, and the like. The treatment can be provided, for example, as an aerosolized spray into the air passing through the airflow distribution system 200. In an embodiment, the treatment can be provided at predetermined intervals. In an embodiment, the treatment can be provided in predetermined amounts. In an embodiment, the treatment dispensed by treatment dispenser 220 can include an anti-pathogen material, for example as the treatment or included in a treatment solution. The anti-pathogen material can include one or more of any suitable materials for destroying or deactivating pathogens in air or on surfaces within the transit vehicle. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, the EPA. The anti-pathogen material can be a material included in an amount deemed safe for use where it may be inhaled, for example by selecting the material and/or the amount of the material based on materials safety standards such as those promulgated by the EPA or any other such suitable standard.

Heat exchanger 225 is a heat exchanger configured to exchange heat between a working fluid (e.g. refrigerant) and the air passing through the airflow distribution system 200. Heat exchanger 225 can be, for example, an evaporator included in a climate control circuit that absorbs heat from the air passing through heat exchanger 225.

Heater 230 can be included in the airflow distribution system 200 to heat air passing through the airflow distribution system 200. Heater 230 can be any suitable heater for a transit HVAC system, such as, for example, an electric heater, a heat exchanger allowing exchange of heat with a fluid at a higher temperature than the air passing through, or the like. The airflow distribution system 200 also includes blower 235, which can be any suitable blower for driving air flow through the airflow distribution system 200.

Air distribution duct 240 is a duct configured to convey air heated or cooled by the airflow distribution system 200 to one or more interior spaces of the climate controlled space of the transit vehicle. Air distribution duct 240 can be any suitable arrangement of one or more ducts providing a path for air heated or cooled by the HVAC system to pass into the one or more interior spaces of the climate controlled space of the transit vehicle. The one or more interior spaces of the climate controlled space can include a driver compartment and a passenger compartment, or a combined internal space used by both driver and passengers. In an embodiment, the air distribution duct can provide air heated or cooled by the HVAC system primarily to the passenger compartment or a passenger portion of the climate controlled space of the transit vehicle. In an embodiment, air distribution duct 240 is configured to supply the air heated or cooled by HVAC system first to the driver of the transit vehicle, for example in a driver section such as driver section 115 described above and shown in Figure 1. In an embodiment, the air distribution duct 240 includes ducting configured to provide air at or closest to the driver's position within the internal space of the transit vehicle.

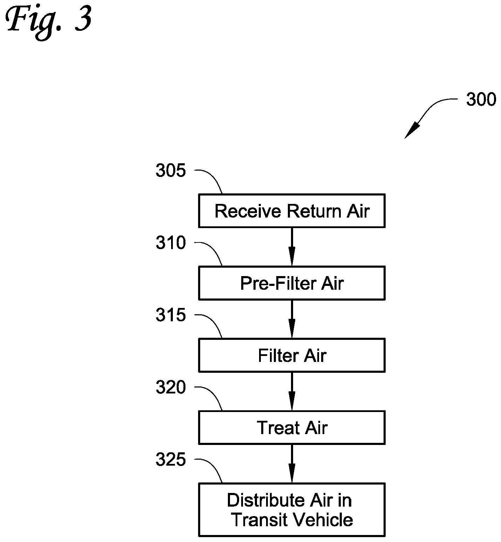

Figure 3 shows a flowchart of a method 300 for controlling pathogen exposure in a transit vehicle. Method 300 can be carried out using an airflow distribution system of the transit vehicle. Method 300 includes receiving return air 305, optionally pre-filtering the air 310, filtering the air 315, optionally treating the air 320, and distributing the air to the interior of the transit vehicle 325.

Return air is received at 305. The return air can be air from the interior of the transit vehicle. The return air can be received at 305 in a return air duct of the airflow distribution system of the transit vehicle. The return air received at 305 is directed to and through the airflow distribution system of the transit vehicle.

Optionally, the air can be pre-filtered at 310. In the optional pre-filtering, air can be passed through a filter positioned upstream of another filter used in the filtering of the air at 315. In an embodiment, the pre-filtering is performed using an auxiliary filter, such as auxiliary filter 210 described above and shown in Figure 2. The air pre-filtering at 310 can be performed using a filter that is more selective than the filter used at 315. The air pre-filtering at 310 can be performed using an auxiliary filter such as auxiliary filter 210 described above and shown in Figure 2. In an embodiment, the filter used for pre-filtering at 310 can include an anti-pathogen material, for example including, being impregnated with, or coated with the anti-pathogen material. The anti-pathogen material can include one or more of any suitable materials for destroying or deactivating pathogens. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, Environmental Protection Agency (EPA). The anti-pathogen material can be a material included in an amount deemed safe for use where it may be inhaled, for example by selecting the material and/or the amount of the material based on materials safety standards such as those promulgated by the EPA or any other such suitable standard. In an embodiment, pre-filtering at 310 can be performed using a filter that is positioned such that it is more readily accessible for servicing or replacement in comparison with the filter used at 315. In an embodiment, the filter used for pre-filtering at 310 can be accessed from within an internal space of the transit vehicle to be serviced or replaced.

The air is then filtered at 315. In an embodiment, filtering the air at 315 includes directing the air to and through a standard air filter for a transit HVAC system. In this embodiment, additional optional steps for reducing pathogen risk, such as the pre-filtering at 310 or the treatment of the air at 320 can be included in the method 300. In an embodiment, the filter used at 315 can include an anti-pathogen material. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, Environmental Protection Agency (EPA). The anti-pathogen material can be a material included in an amount deemed safe for use where it may be inhaled, for example by selecting the material and/or the amount of the material based on materials safety standards such as those promulgated by the EPA or any other such suitable standard.

Optionally, the air can be treated at 320. The air can be treated at 320 using a treatment device such as treatment dispenser 220 described above and shown in Figure 2. In embodiments, treatment of the air at 320 can be performed at any point between receiving the return air at 305 and distributing the air to the interior of the transit vehicle 325. The treatment can be, for example, one or more chemicals selected to treat the air as it passes through the airflow distribution system. The treatment can include air fresheners, anti-microbial or anti-pathogen agents, deodorants, scented materials such as aromatherapy scents, cleaners for HVAC system heat exchangers, and the like. The treatment can be provided at 320 as, for example, an aerosolized spray into the air passing through the airflow distribution system. In an embodiment, the treatment can be provided at 320 at predetermined intervals. In an embodiment, the treatment can be provided at 320 in predetermined amounts. In an embodiment, the treatment provided at 320 can include an anti-pathogen material, for example as the treatment or included in a treatment solution. The anti-pathogen material can include one or more of any suitable materials for destroying or deactivating pathogens in air or on surfaces within the transit vehicle. The anti-pathogen material can include, for example, materials recognized as being effective at destroying or deactivating viruses such as, for example, COVID-19. The recognition of effectiveness can be based, for example, on lists of effective compounds distributed by governmental or standards-setting agencies such as, for example, the EPA. The anti-pathogen material can be a material that is provided in the treatment at 320 in an amount deemed safe for use where it may be inhaled, for example by selecting the material and/or the amount of the material based on materials safety standards such as those promulgated by the EPA or any other such suitable standard.

The air is distributed to the climate controlled space of the transit vehicle at 325. In an embodiment, the return of the air at 325 is a return through standard ducting and/or vents of the airflow distribution system, for example standard ducting and/or vents that supply the air to the passenger portion of the internal space of the transit vehicle. In an embodiment, ducting and/or vents that direct the air to be discharged first at a driver portion of the internal space of the transit vehicle can be used to distribute air at 325. In this embodiment, the driver, who spends the longest continuous time within the transit vehicle, receives air having a relatively lower pathogen content or risk compared to standard arrangements where air comes to the driver after being supplied to and passing through the passenger portion of the internal space of the transit vehicle. At least some of the air distributed to the interior of the transit vehicle 325 can be subsequently taken back in by the airflow distribution system as return air received at 305.

At any point in method 300 following the return air being received at 305 and prior to the distribution of the air at 325, the air can be heated or cooled by the HVAC system. The control of pathogen exposure through method 300 can be independent of heating or cooling operations of the HVAC system. Method 300 can continue to be performed when the HVAC system is providing ventilation only, or when the HVAC system is not actively providing heating or cooling to the air, for example when the temperature in the transit vehicle is at a temperature set point or within a permissible range based on one or more set points for the temperature in the transit vehicle.

### Aspects:

It is understood that any of aspects 1-9 can be combined with any of aspects 10-15.
Aspect 1. An airflow distribution system of a heating, ventilation, and air conditioning (HVAC) system for a transit vehicle, the airflow distribution system comprising:
   a return air duct configured to receive air from an internal space of the transit vehicle;
   a filter configured to remove particulate matter from air passing through the filter;
   at least one sanitizer configured to remove pathogens from air passing through the airflow distribution system;
   a heat exchanger disposed along a path taken by the air passing through the airflow distribution system; and
   an air distribution duct configured to convey the air passing through the airflow distribution system to the interior space of the transit vehicle.
Aspect 2. The airflow distribution system according to aspect 1, further comprising a heater configured to heat air passing through the airflow distribution system.
Aspect 3. The airflow distribution system according to aspect 1 or aspect 2, wherein the air distribution duct is configured to provide the air passing through the HVAC system first to a driver section of the internal space of the transit vehicle.
Aspect 4. The airflow distribution system according to any of aspects 1-3, wherein the sanitizer includes one or more selected from the group consisting of:
   a pre-filter, said pre-filter located upstream of the filter,
   an anti-pathogen material included in or coated on the filter, and
   a treatment dispenser configured to provide a treatment to air passing through the HVAC system.
Aspect 5. The airflow distribution system according to aspect 4, wherein the airflow distribution system includes the pre-filter, and the pre-filter includes the anti-pathogen material.
Aspect 6. The airflow distribution system according to any of aspects 4-5, wherein the HVAC system includes the treatment dispenser, and the treatment dispenser is located between the return air duct and the filter with respect to a flow of the air passing through the HVAC system.
Aspect 7. The airflow distribution system according to any of aspects 4-5 wherein the HVAC system includes the treatment dispenser, and the treatment dispenser is located between the heat exchanger and the air distribution duct with respect to a flow of the air passing through the HVAC system.
Aspect 8. The airflow distribution system according to any of aspects 4-7 wherein the HVAC system includes the treatment dispenser, and the treatment further includes one or more of an air freshener, a deodorant, a scented material, or a cleaner for the heat exchanger.
Aspect 9. The airflow distribution system according to any of aspects 1-8, wherein the HVAC system includes two or more of the sanitizers.
Aspect 10. A method of operating an airflow distribution of a heating, ventilation, and air conditioning (HVAC) system for a transit vehicle, comprising:
   receiving return air;
   reducing pathogens in air passing through the airflow distribution system including the return air, and
   distributing the air passing through the airflow distribution system to an internal space of the transit vehicle.
Aspect 11. The method according to aspect 10, wherein distributing the air passing through the HVAC system through the HVAC system to an internal space of the transit vehicle includes providing the air passing through the HVAC system first to a driver section of the internal space of the transit vehicle.
Aspect 12. The method of according to aspect 10 or aspect 11, wherein the reducing pathogens includes one or more of:
   filtering the air passing through the HVAC system using a pre-filter upstream of a filter of the HVAC system,
   filtering the air passing through the HVAC system using the filter of the HVAC system, wherein the filter of the HVAC system includes or is treated with an anti-pathogen material, and
   providing a treatment to the air passing through the HVAC system, wherein the treatment includes an anti-pathogen material.
Aspect 13. The method according to aspect 12, wherein the reducing pathogens includes filtering the air passing through the airflow distribution system using the pre-filter, and the pre-filter includes the anti-pathogen material.
Aspect 14. The method according to any of aspects 12-13, wherein the reducing pathogens includes providing the treatment to the air passing through the HVAC system, and the treatment further includes one or more of an air freshener, a deodorant, a scented material, or a cleaner for an HVAC system heat exchanger.
Aspect 15. The method according to any of aspects 10-14, wherein the reducing pathogens includes two or more of:
   filtering the air passing through the HVAC system using a pre-filter upstream of a filter of the HVAC system,
   filtering the air passing through the HVAC system using the filter of the HVAC system, wherein
   the filter of the HVAC system includes or is treated with an anti-pathogen material, and
   providing a treatment to the air passing through the HVAC system, wherein the treatment includes an anti-pathogen material.

The examples disclosed in this application are to be considered in all respects as illustrative and not limitative. The scope of the invention is indicated by the appended claims rather than by the foregoing description; and all changes which come within the meaning and range of equivalency of the claims are intended to be embraced therein.

## Claims

1. An airflow distribution system of a heating, ventilation, and air conditioning (HVAC) system for a transit vehicle, the airflow distribution system comprising:
a return air duct configured to receive air from an internal space of the transit vehicle;
a filter configured to remove particulate matter from air passing through the filter;
at least one sanitizer configured to remove pathogens from air passing through the airflow distribution system;
a heat exchanger disposed along a path taken by the air passing through the airflow distribution system; and
an air distribution duct configured to convey the air passing through the airflow distribution system to the interior space of the transit vehicle.

2. The airflow distribution system of claim 1, further comprising a heater configured to heat air passing through the airflow distribution system.

3. The airflow distribution system of any one of claims 1 and 2, wherein the air distribution duct is configured to provide the air passing through the HVAC system first to a driver section of the internal space of the transit vehicle.

4. The airflow distribution system of any one of claims 1-3, wherein the sanitizer includes one or more selected from the group consisting of:
a pre-filter, said pre-filter located upstream of the filter,
an anti-pathogen material included in or coated on the filter, and
a treatment dispenser configured to provide a treatment to air passing through the HVAC system.

5. The airflow distribution system of claim 4, wherein the airflow distribution system includes the pre-filter, and the pre-filter includes the anti-pathogen material.

6. The airflow distribution system of claim 4, wherein the airflow distribution system includes the treatment dispenser, and the treatment dispenser is located between the return air duct and the filter with respect to a flow of the air passing through the airflow distribution system.

7. The airflow distribution system of claim 4, wherein the airflow distribution system includes the treatment dispenser, and the treatment dispenser is located between the heat exchanger and the air distribution duct with respect to a flow of the air passing through the airflow distribution system.

8. The airflow distribution system of claim 4, wherein the airflow distribution system includes the treatment dispenser, and the treatment further includes one or more of an air freshener, a deodorant, a scented material, or a cleaner for the heat exchanger.

9. The airflow distribution system of any one of claims 1-8, wherein the airflow distribution system includes two or more of the sanitizers.

10. A method of operating an airflow distribution of a heating, ventilation, and air conditioning (HVAC) system for a transit vehicle, comprising:
receiving return air;
reducing pathogens in air passing through the airflow distribution system including the return air, and
distributing the air passing through the airflow distribution system to an internal space of the transit vehicle.

11. The method of claim 10, wherein distributing the air passing through the airflow distribution system to the internal space of the transit vehicle includes providing the air passing through the airflow distribution system first to a driver section of the internal space of the transit vehicle.

12. The method of any one of claims 10 and 11, wherein the reducing pathogens includes one or more of:
filtering the air passing through the HVAC system using a pre-filter upstream of a filter of the HVAC system,
filtering the air passing through the HVAC system using the filter of the HVAC system, wherein the filter of the HVAC system includes or is treated with an anti-pathogen material, and
providing a treatment to the air passing through the HVAC system, wherein the treatment includes an anti-pathogen material.

13. The method of claim 12, wherein the reducing pathogens includes filtering the air passing through the airflow distribution system using the pre-filter, and the pre-filter includes the anti-pathogen material.

14. The method of claim 12, wherein the reducing pathogens includes providing the treatment to the air passing through the airflow distribution system, and the treatment further includes one or more of an air freshener, a deodorant, a scented material, or a cleaner for an HVAC system heat exchanger.

15. The method of any one of claims 10-14, wherein the reducing pathogens includes two or more of:
filtering the air passing through the airflow distribution system using a pre-filter upstream of a filter of the airflow distribution system,
filtering the air passing through the airflow distribution system using the filter of the airflow distribution system, wherein the filter of the airflow distribution system includes or is treated with an anti-pathogen material, and
providing a treatment to the air passing through the airflow distribution system, wherein the treatment includes an anti-pathogen material.
